# EUROPÄISCHE PATENTSCHRIFT

(11) **EP 2 181 083 B1**
(45) Veröffentlichungstag und Bekanntmachung des Hinweises auf die Patenterteilung: **12.10.2011**
(21) Anmeldenummer: 08801600.1
(22) Anmeldetag: 18.08.2008
(51) Int. Cl.: C07C 29/151, B01J 8/04, B01J 8/06

(54) **VERFAHREN UND ANLAGE ZUR HERSTELLUNG VON METHANOL**
METHOD AND SYSTEM FOR THE PRODUCTION OF METHANOL
PROCÉDÉ ET INSTALLATION DE PRODUCTION DE MÉTHANOL

(30) Priorität: 29.08.2007 DE 102007040707
(43) Veröffentlichungstag der Anmeldung: 05.05.2010
(73) Patentinhaber: Lurgi GmbH, 60295 Frankfurt am Main (DE)
(72) Erfinder: MÜLLER, Dierk, 61184 Karben (DE); BORMANN, Andreas, 60389 Frankfurt am Main (DE)
(74) Vertreter: KEIL & SCHAAFHAUSEN Patentanwälte
(86) Internationale Anmeldenummer: PCT/EP2008/006759
(87) Internationale Veröffentlichungsnummer: WO 2009/030353

(56) Entgegenhaltungen:
- EP-A- 0 790 226
- EP-A- 1 026 141

## Beschreibung

Die Erfindung betrifft die Herstellung von Methanol aus einem Wasserstoff und Kohlenstoffoxide enthaltenden Synthesegas, wobei man das Synthesegas durch einen ersten, vorzugsweise wassergekühlten Reaktor leitet, in welchem ein Teil der Kohlenstoffoxide katalytisch zu Methanol umgesetzt wird, wobei man das erhaltene, Synthesegas und Methanoldampf enthaltende Gemisch einem zweiten, vorzugsweise gasgekühlten Reaktor zuführt, in welchem ein weitere Teil der Kohlenstoffoxide katalytisch zu Methanol umgesetzt wird, wobei man Methanol von dem Synthesegas abtrennt und wobei man Synthesegas wieder zu dem ersten Reaktor zurückführt.

Ein derartiges Verfahren zur Herstellung von Methanol ist beispielsweise aus der EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem Kupferkatalysator zu Methanol umgesetzt wird. Das in dem Prozess hergestellte Methanol wird von dem zurückzuführenden Synthesegas abgeschieden, welches dann als Kühlmittel durch den gasgekühlten Reaktor hindurchgeführt und auf eine Temperatur von 220 bis 280°C vorgewärmt wird, bevor es in den ersten Synthesereaktor eingebracht wird.

Aus der EP 1 026 141 A1 ist eine Weiterführung dieses Prozesses zur Synthese eines Methanol-Dimethylether-Gemisches aus Synthesegas bekannt. Dabei wird aus dem erwärmten Zufuhrstrom des Synthesegases in den ersten Reaktor noch vor Reaktoreintritt ein Bypassstrom abgezweigt, der der Zufuhrleitung in den zweiten Reaktor beigemischt wird.

Die durch die Thermodynamik vorgegebene maximale Ausbeute von Methanol im gasgekühlten, zweiten Reaktor ist in beiden Verfahren stark vom Temperaturverlauf im Reaktor und dessen Austrittstemperatur abhängig. Ist der wassergekühlte, erste Reaktor mit frischem Katalysator gefüllt, so wird ein Großteil der Kohlenstoffoxide in dem wassergekühlten Reaktor umgesetzt. Der Anteil der im wassergekühlten Reaktor umgesetzten Kohlenstoffoxide sinkt aber mit der Alterung des Katalysators, was dazu führt, dass dann ein Großteil des Gesamtumsatzes an Methanol im gasgekühlten Reaktor stattfindet. Um der Alterung des Katalysators im wassergekühlten Reaktor entgegenzuwirken, wird die Austrittstemperatur aus dem ersten Reaktor und damit die Eintrittstemperatur in den zweiten Reaktor erhöht. Eine Temperaturerhöhung bewirkt jedoch eine Verschiebung des Reaktionsgleichgewichts zu den Edukten im gasgekühlten Reaktor, was eine Verminderung der maximal erreichbaren Methanolausbeute bedeutet.

Aufgabe der Erfindung ist es, auch bei gealtertem Katalysator eine hohe Methanolausbeute des Prozesses zu erreichen.

Diese Aufgabe wird bei einem Verfahren der eingangs genannten Art im Wesentlichen mit den Merkmalen des Anspruchs 1 gelöst. Durch das an dem ersten Reaktor vorbeigeführte Synthesegas, welches vorzugsweise gemeinsam mit dem aus dem wassergekühlten ersten Reaktor abgezogenen Gasgemisch in den zweiten Reaktor eingeführt wird, kann die Temperatur des aus dem ersten Reaktor abgezogenen Gasgemisches abgesenkt werden, so dass der Kohlenstoffumsatz im zweiten Reaktor steigt. Die Temperaturabsenkung wird hierbei durch die Zündtemperatur des Katalysators begrenzt, die bei den herkömmlicherweise verwendeten Kupferkatalysatoren bei etwa 220 °C liegt.

Der an dem ersten Reaktor vorbeigeführte Teilstrom wird aus dem nach der Methanolabtrennung zurückgeführten Synthesegas abgezweigt. Dieses hat nach der Methanolabscheidung üblicherweise eine Temperatur von etwa 60°C, so dass eine wirksame Temperaturabsenkung der in den zweiten Reaktor eintretenden Gasmischung erreicht wird. Es ist jedoch grundsätzlich auch möglich, für den Bypass frisches Synthesegas zu verwenden, welches jedoch aufgrund seiner üblicherweise deutlich höheren Temperatur von etwa 160°C nur eine entsprechend geringere Temperaturabsenkung des in den zweiten Reaktor eingeführten Gasgemisches ermöglicht bzw. einen entsprechend größeren Bypassstrom erfordert.

In Weiterbildung des Erfindungsgedankens liegt der Anteil des an dem ersten Reaktor vorbeigeführten Teilstroms in einem Bereich von 0 bis 20 Vol.-%, vorzugsweise 5 bis 15 Vol.-% und insbesondere bei etwa 10 Vol.-% des zurückgeführten Synthesegases. Hierdurch kann die gewünschte Eintrittstemperatur des Gasgemisches am zweiten Reaktor erreicht werden.

Um der Passivierung des alternden Katalysators im ersten Reaktor entgegenzuwirken, wird die Temperatur im Reaktor über die Erhöhung des Dampfdrucks bewusst angehoben. Dadurch steigt auch die Austrittstemperatur dieses Reaktors. Gemäß einer besonders bevorzugten Ausgestaltung ist daher vorgesehen, dass die Größe des an dem ersten Reaktor vorbeigeführten Teilstroms in Abhängigkeit von der Temperatur des Gasgemischs am Austritt des ersten Reaktors geregelt wird. Dadurch wird eine optimale Anpassung der Eintrittstemperatur des zweiten Reaktors an den Zustand des ersten Reaktors erreicht, so dass eine maximale Methanolausbeute ermöglicht wird.

Der als Bypass an dem ersten Reaktor vorbeigeführte Synthesegas-Recyclingstrom wird zusammen mit frischem Synthesegas dem ersten Reaktor zugeführt, wobei der Anteil des frischen Synthesegases vorzugsweise etwa 15 bis 40 Vol.-% beträgt.

Um eine geeignete Eintrittstemperatur für den ersten Reaktor zu erreichen, wird das Synthesegas als Kühlmittel in den zweiten Reaktor eingeleitet und dadurch vorgewärmt. Zusätzliche Heizeinrichtungen können dadurch vermieden werden. Die Erfindung erstreckt sich auch auf eine Anlage zur Herstellung von Methanol aus einem Wasserstoff und Kohlenstoffoxid enthaltenden Synthesegas, die insbesondere zur Durchführung des oben beschriebenen erfindungsgemäßen Verfahrens geeignet ist und die Merkmale des Anspruchs 6 aufweist.

Um die Eintrittstemperatur (Regelgröße) des zweiten Reaktors auf einen für die maximale Methanolgewinnung geeigneten Wert einstellen zu können, ist erfindungsgemäß ein Regelventil zur Beeinflussung der Größe des durch die Bypassleitung fließenden Gasstroms (Stellgröße) vorgesehen. Die Regelung erfolgt vorzugsweise anhand der Temperatur am Austritt des ersten Reaktors (Messgröße), die über einen Temperatursensor erfasst wird.

Weiterbildungen, Vorteile und Anwendungsmöglichkeiten der Erfindung ergeben sich auch aus der nachfolgenden Beschreibung eines Ausführungsbeispiels und der Zeichnung. Dabei bilden alle beschriebenen und/oder bildlich dargestellten Merkmale für sich oder in beliebiger Kombination den Gegenstand der Erfindung, unabhängig von ihrer Zusammenfassung und den Ansprüchen oder deren Rückbeziehung.

Die einzige Figur zeigt schematisch eine Anlage zur Durchführung des erfindungsgemäßen Verfahrens.

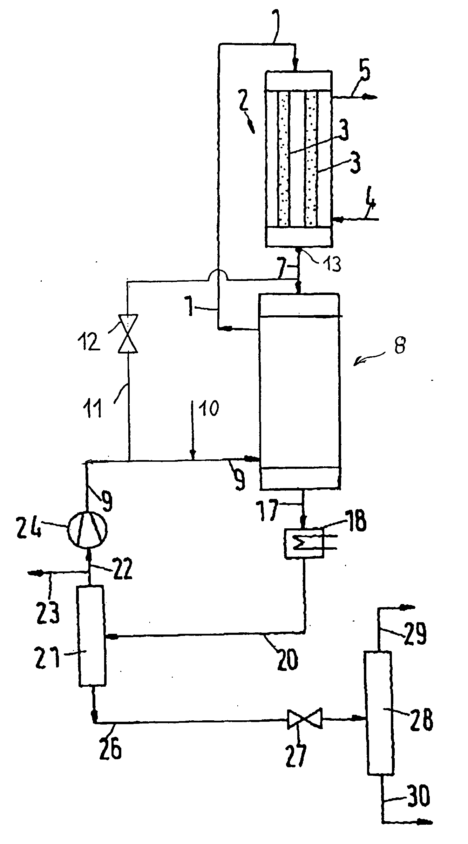

In der in Fig. 1 dargestellten Anlage wird ein Gemisch aus frischem und zurückgeführtem Synthesegas durch eine Leitung 1 in einen ersten Synthese-Reaktor 2 geführt. Dieser erste Reaktor 2 ist ein an sich bekannter Röhrenreaktor, in welchem beispielsweise ein Kupferkatalysator in Röhren 3 angeordnet ist. Als Kühlmittel dient unter erhöhtem Druck siedendes Wasser, das in der Leitung 4 herangeführt wird. Ein Gemisch aus siedendem Wasser und Wasserdampf zieht man in der Leitung 5 ab und führt es zur Energiegewinnung einer nicht dargestellten, an sich bekannten Dampftrommel zu.

In der in Fig. 1 dargestellten Anlage wird ein Gemisch aus frischem und zurückgeführtem Synthesegas durch eine Leitung 1 in einen ersten Synthese-Reaktor 2 geführt. Dieser erste Reaktor 2 ist ein an sich bekannter Röhrenreaktor, in welchem beispielsweise ein Kupferkatalysator in Röhren 3 angeordnet ist. Als Kühlmittel dient unter erhöhtem Druck siedendes Wasser, das in der Leitung 4 herangeführt wird. Ein Gemisch aus siedendem Wasser und Wasserdampf zieht man in der Leitung 5 ab und führt es zur Energiegewinnung einer nicht dargestellten, an sich bekannten Dampftrommel zu.

Das in den ersten Reaktor 2 eintretende Synthesegas ist auf eine Temperatur > 220 °C vorgewärmt, da der Katalysator erst ab dieser Temperatur anspricht. Üblicherweise liegen die Gastemperatur am Eintritt des ersten Reaktors 2 bei 220 bis 280 °C und der Druck im Bereich von 2 bis 12 MPa (20 bis 120 bar), vorzugsweise im Bereich von 3 bis 10 MPa (30 bis 100 bar) und insbesondere im Bereich von 4 bis 10 MPa (40 bis 100 bar). Das Kühlmittel, das man über die Leitung 5 abzieht, hat üblicherweise eine Temperatur im Bereich von 240 bis 280 °C. In dem ersten Reaktor 2 werden in einer exothermen Reaktion je nach Zustand des Katalysators 40 bis 80 % der durch die Leitung 1 in den Reaktor 2 gegebenen Kohlenstoffoxide umgesetzt.

Aus dem ersten Reaktor 2 zieht man über die Leitung 7 ein erstes Gemisch im Wesentlichen bestehend aus Synthesegas und Methanoldampf ab, wobei der Methanolgehalt 4 bis 10 Vol.-%, zumeist 5 bis 8 Vol.-%, beträgt. Dieses Gemisch leitet man in den zweiten Synthesereaktor 8, der beispielsweise ebenfalls als Röhrenreaktor mit einem Kupferkatalysator ausgestaltet ist. Der Katalysator kann wie bei dem ersten Reaktor 2 in den Röhren oder bevorzugt auf der Mantelseite vorgesehen sein.

Als Kühlmedium dient in dem zweiten Reaktor 8 Synthesegas, das über die Leitung 9 mit einer Temperatur von 80 bis 130 °C herangeführt wird. Frisches Synthesegas, das man in einer an sich bekannten, hier nicht dargestellten Anlage erzeugt, wird in der Leitung 10 herangeführt und dem zurückzuführenden Synthesegas zugemischt. Die Temperatur des Kühlgases am Eintritt in den zweiten Reaktor 8 ergibt sich durch das Mischungsverhältnis zwischen rückgeführtem und frischem Synthesegas und wird umso niedriger gewählt je höher die Eintrittstemperatur des ersten Gemischs in den zweiten Reaktor 8 ist. Das als Kühlmittel dienende Synthesegas wird im zweiten Reaktor 8 vorgewärmt und strömt dann durch die Leitung 1 zum ersten Reaktor 2.

Das Synthesegas, das in den ersten Reaktor 2 eintritt, soll Wasserstoff und Kohlenstoffoxide etwa in folgenden Anteilen aufweisen:
H₂ = 40 bis 80 Vol.-%
CO = 3 bis 15 Vol.-% und
CO₂ = 1 bis 10 Vol.-%.

Ein im Wesentlichen Synthesegas und Methanoldampf enthaltendes Produktgemisch (zweites Gemisch) verlässt den zweiten Reaktor 8 durch eine Leitung 17 und strömt durch einen indirekten Kühler 18, wobei Methanol kondensiert wird. Anschließend gibt man das Gemisch durch die Leitung 20 in einen ersten Abscheidebehälter 21, in welchem sich Gase und Flüssigkeit trennen. Die Gase werden durch die Leitung 22 abgezogen, wobei man einen Teil über eine Leitung 23 aus dem Verfahren entfernen kann. Mit Hilfe des Verdichters 24 führt man die Gase als zurückzuführendes Synthesegas (Recyclegas) über die Leitung 9 durch den zweiten Reaktor 8 und nach der hierdurch erfolgten Vorwärmung dann weiter in den ersten Reaktor 2. Hierbei wird über eine Bypassleitung 11 ein Teilstrom des zurückgeführten Synthesegases abgezweigt und an dem ersten Reaktor 2 vorbei direkt dem Eintrittsbereich des zweiten Reaktors 8 zugeführt. Dieser Teilstrom, der üblicherweise eine Temperatur von 50 bis 100°C aufweist, wird in der Leitung 7 mit dem aus dem ersten Reaktor 2 austretenden ersten Gemisch vermischt und senkt dadurch dessen Temperatur ab.

Die Größe des durch die Bypassleitung 11 geführten Teilstromes wird über ein insbesondere in der Bypassleitung 11 vorgesehenes Regelventil 12 geregelt, wobei die Regelung auf der Basis der über einen Temperatursensor 13 am Austritt des ersten Reaktors 2 erfassten Austrittstemperatur geregelt wird. Erfindungsgemäß wird die Größe des Teilstroms so eingestellt, dass er beginnend bei 0 Vol.-%, also ohne Bypassstrom, (frischer Katalysator im ersten Reaktor 2) bis zu einem Anteil von 15 oder 20 %, vorzugsweise aber etwa 10 Vol.-% des über die Leitung 9 zurückgeführten Synthesegasstromes (bei gealtertem Katalysator im ersten Reaktor 2) erhöht wird.

Aus dem ersten Abscheidebehälter 21 zieht man über eine Leitung 26 Methanol enthaltende Flüssigkeit ab und führt die Flüssigkeit durch ein Entspannungsventil 27 zu einem zweiten Abscheidebehälter 28. Hieraus wird über eine Leitung 29 ein Restgas abgezogen, während man über die Leitung 30 Rohmethanol erhält, das nun in nicht dargestellter, an sich bekannter Weise destillativ gereinigt wird.

Es versteht sich, dass der Aufbau der Reaktoren 2, 8 als solchen nicht auf die oben beschriebenen Varianten beschränkt ist. Vielmehr sind auch Abwandlungen dieser Reaktoren möglich, beispielsweise so wie es in der EP 0 790 226 B1 beschrieben ist.

### Beispiel

Über die Leitung 9 wird dem zweiten Reaktor 8 eine Mischung aus rückgeführtem Synthesegas und frischem Synthesegas als Kühlgas zugeführt. Das zurückgeführte Synthesegas hat hierbei eine Temperatur von etwa 60 °C, während das frische Synthesegas eine Temperatur von etwa 160 °C aufweist. Das Mischungsverhältnis wird zunächst so gewählt, dass sich eine Mischtemperatur von etwa 120 °C einstellt. Erhöht sich die Gasaustrittstemperatur des ersten Reaktors 2, so kann die Mischungstemperatur des Kühlgases am unteren Eintritt in den zweiten Reaktor 8 bspw. auch auf 90 °C abgesenkt werden, um die höhere Temperatur des Synthesegases zu kompensieren. Nach Durchlaufen des gasgekühlten Reaktors 8 als Kühlmittel steigt die Temperatur des Synthesegases, das über die Leitung 1 mit einem Druck von etwa 8 MPa (80 bar) in den wassergekühlten ersten Reaktor 2 eingeführt wird, auf etwa 230 bis 240 °C, jedenfalls größer als 220 °C. Aufgrund der exothermen Reaktion und gleichzeitigen Kühlung in dem ersten Reaktor 2 tritt das erste Gemisch aus Synthesegas und Methanoldampf mit einer Temperatur von 220 bis 230 °C aus dem ersten Reaktor 2 aus. Über den ersten Reaktor 2 stellt sich ein Druckverlust von etwa 0,2 MPa (2 bar) ein. Mit Alterung des Katalysators kann sich diese Austrittstemperatur auf bspw. 270 °C erhöhen. Durch Zumischung eines entsprechend der Gasaustrittstemperatur des ersten Reaktors 2 steigenden Anteils von bis 10 % des zurückgeführten Synthesegases über die Bypassleitung 11 wird die Temperatur des über die Leitung 1 in den gasgekühlten zweiten Reaktor 8 eintretenden Gasgemischs auf etwa 230 bis 240 °C abgesenkt. Nach Durchlaufen des zweiten Reaktors 8 hat das zweite Gemisch aus Methanoldampf und Synthesegas eine Temperatur von etwa 220 °C und einen Druck von etwa 7,5 MPa (75 bar).

Auch bei Steigerung des Austrittstemperatur des ersten Reaktors 2 kann somit die Eintrittstemperatur des Synthesegases in den zweiten Reaktor 8 so eingestellt werden, dass eine maximale Methanolausbeute erreicht wird.

### Bezugszeichenliste:

- 1: Leitung (erste Rückführleitung)
- 2: erster Reaktor
- 3: Röhren
- 4: Leitung
- 5: Leitung
- 7: Leitung
- 8: zweiter Reaktor
- 9: Leitung (zweite Rückführleitung)
- 10: Leitung
- 11: Bypassleitung
- 12: Regelventil
- 13: Temperatursensor
- 17: Leitung
- 18: Kühler
- 20: Leitung
- 21: Abscheidebehälter
- 22: Leitung
- 23: Leitung
- 24: Verdichter
- 26: Leitung
- 27: Entspannungsventil
- 28: zweiter Abscheidebehälter
- 29: Leitung
- 30: Leitung

## Patentansprüche

1. Verfahren zur Herstellung von Methanol aus einem Wasserstoff und Kohlenstoffoxide enthaltenden Synthesegas, wobei man das Synthesegas durch einen ersten, vorzugsweise wassergekühlten Reaktor leitet, in welchem ein Teil der Kohlenstoffoxide katalytisch zu Methanol umgesetzt wird, wobei man das erhaltene, Synthesegas und Methanoldampf enthaltende Gemisch einem zweiten, vorzugsweise gasgekühlten Reaktor zuführt, in welchem ein weiterer Teil der Kohlenstoffoxide zu Methanol umgesetzt wird, wobei man Methanol von dem Synthesegas abtrennt, wobei man Synthesegas wieder zu dem ersten Reaktor zurückführt, und wobei ein Teilstrom des Synthesegases an dem ersten Reaktor vorbeigeführt und in den zweiten Reaktor eingeleitet wird, **dadurch gekennzeichnet, dass** der an dem ersten Reaktor vorbeigeführte Teilstrom aus dem nach der Methanolabtrennung zurückgeführten Synthesegas abgezweigt wird.

2. Verfahren nach Anspruch 1, **dadurch gekennzeichnet, dass** der Anteil des an dem ersten Reaktor vorbeigeführten Teilstroms in einem Bereich von 0 bis 20 Vol.-%, vorzugsweise 5 bis 15 Vol.-%, des zurückgeführten Synthesegases liegt.

3. Verfahren nach einem der vorhergehenden Ansprüche, **dadurch gekennzeichnet, dass** die Größe des an dem ersten Reaktor vorbeigeführten Teilstroms in Abhängigkeit von der Temperatur des Gasgemischs am Austritt des ersten Reaktors geregelt wird.

4. Verfahren nach einem der vorhergehenden Ansprüche, **dadurch gekennzeichnet, dass** das zurückgeführte Synthesegas zusammen mit frischem Synthesegas dem ersten Reaktor zugeführt wird, und dass der Anteil des frischen Synthesegases 15 bis 40 Vol.-% beträgt.

5. Verfahren nach einem der vorhergehenden Ansprüche, **dadurch gekennzeichnet, dass** man das Synthesegas als Kühlmittel in den zweiten Reaktor einleitet und dadurch vorwärmt.

6. Anlage zur Herstellung von Methanol aus einem Wasserstoff und Kohlenstoffoxide enthaltenden Synthesegas, insbesondere zur Durchführung eines Verfahrens nach einem der vorhergehenden Ansprüche, mit einen ersten, vorzugsweise wassergekühlten Reaktor (2), in welchem ein Teil der Kohlenstoffoxide katalytisch zu Methanol umgesetzt wird, mit einem zweiten, vorzugsweise gasgekühlten Reaktor (8), welchem man das aus dem ersten Reaktor (2) erhaltene Gasgemisch über eine Leitung (7) zuführt und in welchem ein weiterer Teil der Kohlenstoffoxide zu Methanol umgesetzt wird, mit einer Abscheideeinrichtung (21) zur Abtrennung des Methanols von dem Synthesegas, mit einer ersten Rückführleitung (1) zur Rückführung von Synthesegas zu dem ersten Reaktor (2), mit einer zweiten Rückführleitung (9) zur Rückführung von Synthesegas von der Abscheideeinrichtung (21) zu dem zweiten Reaktor (2), und mit einer Bypassleitung (11) für Synthesegas, die an dem ersten Reaktor (2) vorbei zu dem Eintritt des zweiten Reaktors (8) führt, **dadurch gekennzeichnet, dass** die Bypassleitung (11) von der zweiten Rückführleitung (9) abzweigt.

7. Anlage nach Anspruch 6, **gekennzeichnet durch** ein Regelventil (12) zur Beeinflussung der Größe des **durch** die Bypassleitung (11) fließenden Gasstroms.

8. Anlage nach Anspruch 6 oder 7, **gekennzeichnet durch** einen Temperatursensor (13) am Ausgang des ersten Reaktors (2).

## Claims

1. A method for the production of methanol from a synthesis gas containing hydrogen and carbon oxides, the synthesis gas being guided through a first, preferably water-cooled reactor in which some of the carbon oxides are converted catalytically into methanol, the mixture obtained containing synthesis gas and methanol vapour being fed to a second, preferably gas-cooled reactor in which some further carbon oxides are converted into methanol, methanol being separated from the synthesis gas, synthesis gas again being fed back to the first reactor, and a partial flow of the synthesis gas bypassing the first reactor and being introduced into the second reactor, **characterised in that** the partial flow bypassed past the first reactor is branched off from the synthesis gas fed back after the methanol separation.

2. The method according to claim 1, **characterised in that** the proportion of the partial flow bypassed past the first reactor lies in a range of 0 to 20 vol. %, preferably 5 to 15 vol. % of the synthesis gas fed back.

3. The method according to any one of the preceding claims, **characterised in that** the size of the partial flow bypassed past the first reactor is adjusted as a function of the temperature of the gas mixture at the discharge of the first reactor.

4. The method according to any one of the preceding claims, **characterised in that** the synthesis gas fed back is fed to the first reactor together with fresh synthesis gas, and **in that** the proportion of fresh synthesis gas is 15 to 40 vol. %.

5. The method according to any one of the preceding claims, **characterised in that** the synthesis gas is introduced into the second reactor as a coolant and is thus preheated.

6. A system for producing methanol from a synthesis gas containing hydrogen and carbon oxides, in particular for carrying out a method according to any one of the preceding claims, comprising a first, preferably water-cooled reactor (2) in which some of the carbon oxides are converted catalytically into methanol, comprising a second, preferably gas-cooled reactor (8) to which the gas mixture obtained from the first reactor (2) is fed via a line (7) and in which some further carbon oxides are converted into methanol, comprising a separation means (21) for separating the methanol from the synthesis gas, comprising a first return line (1) for feeding synthesis gas back to the first reactor (2), comprising a second return line (9) for feeding synthesis gas from the separation means (21) back to the second reactor (2), and comprising a bypass line (11) for synthesis gas which bypasses the first reactor (2) and leads to the inlet of the second reactor (8), **characterised in that** the bypass line (11) branches off form the second return line (9).

7. The system according to claim 6, **characterised by** a control valve (12) for influencing the size of the gas flow flowing through the bypass line (11).

8. The system according to either claim 6 or claim 7, **characterised by** a temperature sensor (13) at the outlet of the first reactor (2).

## Revendications

1. Procédé de production de méthanol à partir d'un gaz de synthèse contenant de l'hydrogène et des oxydes de carbone, dans lequel on conduit le gaz de synthèse à travers un premier réacteur, de préférence refroidi par eau, dans lequel une partie des oxydes de carbone est transformé par catalyse en méthanol, le mélange obtenu contenant du gaz de synthèse et de la vapeur de méthanol étant conduit vers un deuxième réacteur, de préférence refroidi par gaz, dans lequel une autre partie des oxydes de carbone est transformée en méthanol, le méthanol étant séparé du gaz de synthèse, le gaz de synthèse étant reconduit vers le premier réacteur et un flux partiel du gaz de synthèse étant conduit pour passer devant le premier réacteur et introduit dans le deuxième réacteur, **caractérisé en ce que** le flux partiel conduit pour passer devant le réacteur est dérivé du gaz de synthèse reconduit après la séparation de méthanol.

2. Procédé selon la revendication 1, **caractérisé en ce que** la part du flux partiel conduit pour passer devant le premier réacteur est de l'ordre de 0 à 20 % en volume, de préférence de 5 à 15 % en volume du gaz de synthèse reconduit.

3. Procédé selon l'une quelconque des revendications précédentes, **caractérisé en ce que** la grandeur du flux partiel conduit pour passer devant le premier réacteur est réglée en fonction de la température du mélange gazeux à la sortie du premier réacteur.

4. Procédé selon l'une quelconque des revendications précédentes, **caractérisé en ce que** le gaz de synthèse reconduit est amené vers le premier réacteur ensemble avec du gaz de synthèse neuf et **en ce que** la part du gaz de synthèse neuf est de 15 à 40 % en volume.

5. Procédé selon l'une quelconque des revendications précédentes, **caractérisé en ce qu'**on introduit le gaz de synthèse en tant qu'agent réfrigérant dans le deuxième réacteur et **en ce qu'**on le préchauffe de ce fait.

6. Installation de production de méthanol à partir d'un gaz de synthèse contenant de l'hydrogène et des oxydes de carbone, notamment pour la réalisation d'un procédé selon l'une quelconque des revendications précédentes, avec un premier réacteur (2), de préférence refroidi par eau dans lequel une partie des oxydes de carbone est transformée par catalyse en méthanol, avec un deuxième réacteur (8), de préférence refroidi par gaz vers lequel on amène le mélange gazeux obtenu à partir du premier réacteur (2) via un conduit (7) et dans lequel une autre partie des oxydes de carbone est transformée en méthanol, avec un système séparateur (21) pour séparer le méthanol du gaz de synthèse, avec un premier conduit de retour (1) pour reconduire le gaz de synthèse vers le premier réacteur (2), avec un deuxième conduit de retour (9) pour reconduire le gaz de synthèse du système séparateur (21) vers le deuxième réacteur (2), et avec un conduit de dérivation (11) pour le gaz de synthèse, qui en passant devant le premier réacteur (2) conduit vers l'entrée du deuxième réacteur (8), **caractérisée en ce que** le conduit de dérivation (11) dérive du deuxième conduit de retour (9).

7. Installation selon la revendication 6, **caractérisée par** une soupape de réglage (12) pour influencer la grandeur du flux de gaz s'écoulant à travers le conduit de dérivation (11).

8. Installation selon la revendication 6 ou 7, **caractérisée par** un capteur de température (13) à la sortie du premier réacteur (2).
